(19) Europäisches Patentamt European Patent Office Office européen des brevets

(11) **EP 2 278 952 B1**

(12) **EUROPÄISCHE PATENTSCHRIFT**

(45) Veröffentlichungstag und Bekanntmachung des Hinweises auf die Patenterteilung:
**11.12.2019 Patentblatt 2019/50**

(21) Anmeldenummer: **09753794.8**

(22) Anmeldetag: **13.05.2009**

(51) Int Cl.:
*A61K 8/25* (2006.01)  *A61K 8/37* (2006.01)
*A61Q 19/10* (2006.01)  *C11D 1/28* (2006.01)
*C11D 1/29* (2006.01)  *C11D 1/72* (2006.01)
*C11D 1/83* (2006.01)  *C11D 3/12* (2006.01)
*C11D 3/14* (2006.01)  *C11D 3/20* (2006.01)

(86) Internationale Anmeldenummer:
**PCT/EP2009/055772**

(87) Internationale Veröffentlichungsnummer:
**WO 2009/144139 (03.12.2009 Gazette 2009/49)**

(54) **HAUT- UND HANDREINIGUNGSMITTEL**

SKIN AND HAND CLEANSERS

PRODUITS NETTOYANTS POUR LA PEAU ET LES MAINS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **30.05.2008 DE 102008026051**

(43) Veröffentlichungstag der Anmeldung:
**02.02.2011 Patentblatt 2011/05**

(73) Patentinhaber: **Deb IP Limited**
**Denby, Derbyshire DE5 8JZ (GB)**

(72) Erfinder:
• **ALLEF, Petra**
  **47807 Krefeld (DE)**

• **VEEGER, Marcel**
  **47574 Goch (DE)**
• **HEMMING, Markus**
  **46045 Oberhausen (DE)**

(74) Vertreter: **Elsy, David et al**
**Withers & Rogers LLP**
**4 More London Riverside**
**London SE1 2AU (GB)**

(56) Entgegenhaltungen:
**EP-A- 0 141 410     EP-A- 0 635 260**
**EP-B- 1 504 081     WO-A-01/85103**
**DE-A1- 4 335 933     US-A- 5 444 096**
**US-A- 5 824 327**

## Beschreibung

Die Erfindung betrifft wasserarme bzw. wasserfreie Haut- und Handreinigungsmittel, insbesondere zur Entfernung von extremen Hautverschmutzungen.

Haut- und Handreinigungsmittel werden in der Industrie umfangreich eingesetzt, insbesondere dort, wo hartnäckige Verschmutzungen auftreten, die durch Lacke, Fette, Öle, Schmierstoffe, Metallstäube, Graphit, Ruß u. ä. hervorgerufen werden. Solche Reinigungsmittel sind insbesondere als sogenannte Grobhandreiniger bekannt (vgl. H. Tronnier, J. Kresken, K. Jablonski, B. Komp, "Haut und Beruf", Grosse Verlag, Berlin, S.75-108 [1989]). Üblicherweise handelt es sich hierbei um Zubereitungen, die ein Abrasivum, Tensid/Tensidgemische, Verdickungsmittel sowie gegebenenfalls Hilfsstoffe zur Regulierung der Konsistenz, von Aussehen, Geruch und Stabilität, wie Pigmente, Duftstoffe, Stabilisatoren und Konservierungsmittel, enthalten. Für besonders hartnäckige Verschmutzungen gibt es Produkte, bei denen die Verwendung der oben genannten Inhaltsstoffe nicht ausreicht.

Diesen Zubereitungen werden dann organische Lösungsmittel zugesetzt, wie z. B. aliphatische Koh- lenwasserstoffe, Terpene, Carbonsäureester vom Typ Dimethyladipat, Dimethylglutarat, Dimethylsuccinat (DBE) und Di-n-butyladipat bzw. Di-isopropyladipat, wie sie in der DE 43 35 933 A1 beschrieben worden sind.

Darüber hinaus ist hier auf die im Markt erhältlichen sogenannten "waterless cleaner" hinzuweisen, deren gute Reinigungswirkung vornehmlich auf den vorgenannten organischen Lösemitteln, insbesondere Benzine, Kerosine, kurzkettige Paraffinöle, beruhen.

Im Hinblick auf das große Anwendungsspektrum von Grobhandreinigern, insbesondere im industriellen Bereich bzw. der Tatsache, dass Haut- und Handreinigungsverschmutzungen, wenn sie in diesem Bereich auftreten, vielfach besonders hartnäckig sein können und daher der Reinigung mit konventionellen Hautreinigungsmitteln meist nicht zugänglich sind, beispielsweise in der Lacke verarbeitenden Industrie, besteht weiterhin ein Bedarf an Haut- bzw. Handreinigungsmitteln, die eine vergleichbare Reinigungswirkung zeigen, wie die im Stand der Technik bekannten Produkte, beispielsweise DBE -enthaltenden Haut- und Handreinigungsmittel. Solche Mittel sind hinsichtlich ihrer Reinigungswirkung im sogenannten "Grobhandreinigerbereich", d.h. als Mittel zur Entfernung von extremen Verschmutzungen hinsichtlich ihrer Reinigungswirkung quasi als Standardreiniger anzusehen mit der Folge, dass Haut- und Handreinigungsmittel ohne DBE als hautreinigungsverstärkende Komponente eine zumindest qualitativ vergleichbare hautreinigende Wirkung aufweisen müssen, um für ihren Bestimmungszweck verbraucherseitig Akzeptanz zu finden.

Trotz der vorgenannten Tatsache, dass Carbonsäureester vom Typ Dimethyladipat, Dimethylglutarat, Dimethylsuccinat (DBE) und Di-n-butyladipat bzw. Di-isopropyladipat eine hervorragende Wirksamkeit als Reinigungsverstärker in Haut- und Handreinigungsmitteln aufweisen, ist die Verfügbarkeit dieses kosmetischen Rohstoffes im Markt begrenzt bzw. unterliegt vielfach großen Schwankungen, was natürlich unmittelbar Einfluss auf die Herstellungskosten der Endprodukte hat. Darüber hinaus hat sich gezeigt, dass Haut- und Handreinigungsmittelformulierungen, die DBE als Reinigungsverstärker enthalten, vielfach zusätzlich zu stabilisieren sind, um verkehrsfähige Produkte zu erhalten. Dies ist mit einer zusätzlichen Belastung der Endprodukte von der Kostenseite verbunden. Daher wäre es vorteilhaft, sowohl Haut- und Handreinigungsmittelformulierungen, die DBE als die Hautreinigung verstärkende Komponente enthalten und die in einfacher Weise stabilisiert sind, einsetzen zu können, als auch in gleicher Weise stabilisierte Haut- und Handreinigungsmittelformulierungen, die eine Alternative zu den oben genannten Carbonsäureestern vom DBE-Typ als Reinigungskraftverstärker enthalten.

So werden beispielsweise in der EP 1 504 081 B1 Haut- und Handreinigungsmittel auf Basis von Fettsäuremethylester beschrieben. Nachteilig an den in diesem Dokument beschriebenen Haut- und Handreinigungsmitteln ist, dass diese Haut- und Handreinigungsmittel 10 bis 80 Gew.-% Wasser enthalten. Dies führt nicht nur zu einer verminderten Reinigungsleistung, sondern es besteht die Gefahr der Hydrolyse und der erhöhten Oxidation, insbesondere der Methylester in diesen wässrigen Formulierungen, was mit einer Verminderung der Lagerfähigkeit solcher Haut- und Handreinigungsmittel einhergeht.

Versuche, die zum Ziel hatten, ausgehend von der EP 1 504 081 B1 wasserarme Formulierungen, d.h. Formulierungen mit einem Wassergehalt < 10 Gew.-% herzustellen, führten zu instabilen Produkten bzw. die erhaltenen Produkte bildeten keine Fließrenze aus, um bei einem optionalen Einsatz von Reibepartikeln in den Haut- und Handreinigungsmittel ein Absetzen derselben in der Formulierung zu verhindern, d.h. eine stabile Einarbeitung von Reibepartikeln ist deutlich erschwert bzw. unmöglich.

WO 0185103 offenbart wasserfreie Hautreinigungsmittel, vorzugsweise in Form von Gelen, welche mit Wasser nicht mischbare geschmeidigmachende Öle; Ölgeliermittel, die wasserunlöslich und ölunlöslich sind; Emulgatoren, die in situ auf der Haut eine Emulsion bilden, wenn das Hautreinigungsmittel während des Gebrauchs allmählich mit einer relativ geringen Menge Wasser in Kontakt gebracht wird; und wasserlösliches, im wesentlichen kristallines Schleifmaterial, das in dem wasserfreien Hautreinigungsmittel unlöslich ist, umfassen. Die wasserfreien Hautreinigungsgele entfernen auf kosmetisch ästhetische Weise ölige und wasserlösliche Verschmutzungen von der Haut. Bevorzugte Hautreinigungsgele bilden in situ auf der Haut während des Gebrauchs Reinigungsemulsionen, die mit Wasser von der Haut entfernt werden können.

EP 0 635 260, EP 0 141 410, US 5 824 327 und US 5 444 096 offenbaren Zusammensetzungen, die einen Alkylester oder Diester und eine hydrophile, pyrogene Kieselsäure enthalten.

Aufgabe war es daher, wasserarme bzw. wasserfreie Haut- bzw. auch Handreinigungsmittel, insbesondere zur Reinigung von extremen Haut- und Handreinigungsverschmutzungen mit einem Wassergehalt < 10 Gew.-%, bezogen auf das Gesamtprodukt, bereitzustellen, die unter Ausbildung einer Fließgrenze, um bei Einsatz von Reibepartikeln in den Haut- und Handreinigungsmittel ein Absetzen derselben in der Formulierung zu verhindern, nicht nur eine vergleichbare Reinigungswirkung wie die im Handel erhältlichen aufweisen, sondern auch produktionstechnisch einfach herstellbar sind bzw. diese Haut- und Handreinigungsmittel sollten einen Flammpunkt > 100°C und einen Dampfdruck bei 20°C > 0,01 hPa aufweisen, wobei die Haut- und Handreinigungsmittel dergestalt in einfacher Weise stabilisiert sind, dass sie als homogene und stabile Endprodukte eine gute Lagerfähigkeit aufweisen.

Die Aufgabe wurde überraschend gelöst durch ein wasserfreies bzw. wasserarmes Haut- und Hand- reinigungsmittel, das die Komponenten

a.) mindestens einen Alkylester und/oder Diester

b.) 1 bis 40 Gew.-% mindestens ein Tensid ausgewählt aus der Gruppe der Fettalkoholethoxylate, Fettalkoholethersulfate und Salze sulfierter und/oder sulfonierter Fettsäuren,

c.) 0,5 bis 10 Gew.-%, bezogen auf die Gesamtzusammensetzung, mindestens eines Thixotropiermittels, ausgewählt aus organophilen und/oder hydrophoben Schichtsilikaten, und > 0,1 Gew.-%, bezogen auf die Gesamtzusammensetzung, mindestens einer hydrophilen, pyrogenen Kieselsäure,

d.) 5 bis 30 Gew.-% eines oder mehrerer Abrasiva, ausgewählt aus Sand, Bimsmehl, Calciumcarbonat, Kaolin, Kunststoffreibemittel auf der Basis von Polyethylen oder Polyurethan, Reibemittel auf der Basis von natürlichen Kern-, Hülsen- und/oder Schalenmehlen, oder Gemische dieser Schalen- und Kernmehle, und/oder Perlen aus Wachsen, und/oder wasserquellbare Feststoffteilchen von organischen Polymeren natürlichen und/oder synthetischen Ursprungs,

e.) 0 bis 5 Gew.-% mindestens einen physiologisch verträglichen Kohlensäureester,

f.) 0 bis < 10 Gew.-% Wasser,

g.) gegebenenfalls eines oder mehrere viskositätsbildende Mittel,

h.) gegebenenfalls weitere kosmetische Hilfs-, Zusatz- und/oder Wirkstoffe,

wobei die Summe der Komponenten a.) bis h.) 100 Gew.-%, bezogen auf die Zusammensetzung des Reinigungsmittels, ergibt, aufweist.

Es war völlig überraschend, dass stabile und lagerfähige Haut- und Handreinigungsmittel auf Basis von Fettsäurealkylestern mit einem Wassergehalt < 10 Gew.-%, bezogen auf die Gesamtzusammensetzung, erhalten werden können, wenn diese Mittel eine synergistische Kombination von mindestens einem Thixotropiermittel mit mindestens einer hydrophilen, pyrogenen Kieselsäure als Komponente aufweisen.

Die erfindungsgemäß einzusetzenden Alkylester der Komponente a.) können synthetisch hergestellte Fettsäurealkylester sein, beispielsweise als Umsetzungsprodukte von Fettsäuren mit niederen aliphatischen Alkoholen oder alternativ auch im Wege der Umesterung von natürlichen oder synthetischen Fetten und Ölen gewonnen werden, wobei bei Fettsäurealkylestern, die von natürlich vorkommenden Ölen stammen, diese für den kosmetischen Einsatz entsprechend industriell aufbereitet bzw. gereinigt sind. Fettsäurealkylester der Komponente a.) weisen beispielsweise die allgemeine Formel (I), nämlich

$$\mathbf{R^1CO\text{-}OR^2} \qquad (I)$$

auf, in der $\mathbf{R^1CO}$ für einen linearen oder verzweigten, gesättigten und/oder ungesättigten Acylrest mit 6 bis 22, insbesondere 8 bis 22, vorzugsweise 12 bis 22 Kohlenstoffatomen und $\mathbf{R^2}$ für einen Alkylrest mit 1 bis 8 Kohlenstoffatomen, insbesondere für einen Alkylrest mit 1 bis 4 Kohlenstoffatomen und besonders bevorzugt für einen Methylrest steht. Bevorzugte synthetische Fettsäurealkylester sind hierbei beispielsweise synthetische Methyl-, Ethyl-, Propyl-, Isopropylester, n-Butyl-, Isobutyl-, tert- Butyl sowie die entsprechenden Pentyl- und Hexylester, insbesondere auch Fettsäure-2-ethylhexylester. Erfindungsgemäß bevorzugte Fettsäuremethylester, Fettsäure-isopropylester und Fettsäure-2-ethylhexylester sind von der Fa. Cognis unter dem Handelsnamen TEXAPRINT® erhältlich.

Weitere typische Beispiele sind die Methyl-, Ethyl-, Propyl- oder Butylester von Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Linolsäure, Linolensäure, Gadoleinsäure, Arachidonsäure, Behensäure und Erucasäure sowie deren technischen Gemischen.

Insbesondere werden erfindungsgemäß auch solche Fettsäureester eingesetzt, die sich von sogenannten "Pflanzenölen" ableiten lassen, nämlich die durch Veresterung von beispielsweise Soja-, Sonnenblumen-, Kokos- oder Rapsöl mit einem Alkohol erhältlichen Pflanzenölester. Besonders bevorzugt sind hierbei die Fettsäuremethylester als Komponente a.), die beispielsweise unter dem Namen Edenor ME SU ebenfalls von der Firma Cognis bezogen werden können.

Desweiteren können die Alkylester der Komponente a.) Diester wie beispielsweise Dimethyladipat, Dimethylglutarat, Dimethylsuccinat (DBE), Di-n-butyladipat, Di-isopropyladipat bzw. Dimethyl-2-methyl-glutarate sein. Sie weisen beispielsweise die allgemeine Formel (ii) auf, nämlich

$$R^1OOC\text{-}X\text{-}COOR^2 \qquad (ii)$$

auf, in der X für einen linearen oder verzweigten, gesättigten und/oder ungesättigten Alkylrest mit 0 bis 20, vorzugsweise 0 bis 6 Kohlenstoffatomen und $R^1$ und $R^2$ für einen Alkylrest mit 1 bis 8 Kohlenstoffatomen, insbesondere für einen Methylrest steht.

Die Alkylester und/oder Diester der Komponente a.) können allein oder als Mischungen eingesetzt werden, wobei die Methylester besonders bevorzugt sind.

Die erfindungsgemäßen Haut- und Handreinigungsmittel enthalten vorzugsweise 5 bis 90 Gew.-%, bezogen auf die Gesamtzusammensetzung, vorzugsweise 10 bis 65 Gew.-% bzw. besonders bevorzugt 25 bis 65 Gew.-% Alkylester und/oder Diester der Komponente a.). Die als Komponente b.) einsetzbaren Fettalkoholethoxylate weisen vorzugsweise die allgemeine Formel

$$R\text{-}O\text{-}(CH2\text{-}CH2\text{-}O)nH$$

auf, wobei
R = gesättigter, ungesättigter, verzweiger oder unverzweigter Alkylrest, n = ganze Zahl von 1 bis 11 sind.

Vorzugsweise werden als gesättigter, ungesättigter, verzweiger oder unverzweigter Alkylrest R = $C_6$ bis $C_{18}$, insbesondere $C_{10}$ bis $C_{16}$ und besonders bevorzugt $C_{11}$ bis $C_{14}$ verwendet, wobei bevorzugt n = 3 bis 6, insbesondere n = 5 bis 7 ist.

Bei den auswählbaren Fettalkoholethersulfaten der Komponente b) handelt es sich insbesondere um solche der allgemeinen Formel

$$R\text{-}O\text{-}(CH2\text{-}CH2\text{-}O)_nSO_3X$$

mit R = einem $C_8$-$C_{18}$, bevorzugt $C_{11}$-$C_{14}$ gesättigten oder ungesättigten, verzweigten oder unverzweigten Alkylrest, n = einer ganzen Zahl von 1 bis 6, vorzugsweise 1 bis 4, und X = $Na^+$, $NH_4^+$ oder $Mg^{2+}$, wobei Natriumlaurylethersulfat (mit R = $C_{12}$, n = 2-3 und X = $Na^+$) besonders bevorzugt wird.

Als Salze von sulfierten und/oder sulfonierten Fettsäuren der Komponente b.) werden erfindungsgemäß Alkali-oder Erdalkalisalze von $C_8$-$C_{30}$, bevorzugt $C_{10}$-$C_{22}$ Fettsäuren, besonders bevorzugt Rizinusölsulfate, insbesondere $Na^+$ oder $NH_4^+$ Sulfate verwendet. Solche Rizinusölsulfonate sind beispielsweise unter den Marken Monobrilliantöl® (Evonik Stockhausen GmbH, Krefeld) oder Standapol SCO® (Cognis Deutschland GmbH & Co. KG, Düsseldorf) erhältlich.

In den erfindungsgemäßen Haut- und Handreinigungsmitteln sind 1 bis 40 Gew.-%, bevorzugt 5 bis 30 und besonders bevorzugt 10 bis 30 Gew.-% der Komponente b), vorzugsweise Fettalkoholethoxylate, bezogen auf die Zusammensetzung des Reinigungsmittels, enthalten. In einer bevorzugten Ausführungsform enthalten die erfindungsgemäßen Haut- und Handreinigungsmittel 5 bis 35 Gew.-%, bezogen auf die Zusammensetzung des Reinigungsmittels, Laureth-6 als Fettalkoholethoxylat auf. Erfindungswesentlich ist, dass die erfindungsgemäßen Haut- und Handreinigungsmittel zwingend die Komponente c.) aufweisen, nämlich eine Kombination bestehend aus mindestens einem Thixotropiermittel und mindestens einer hydrophilen, pyrogenen Kieselsäure.

Erfindungsgemäß wird unter einem Thixotropiermittel ein Mittel verstanden, das geeignet ist, die Fließgrenze eines dispersen Systems wie z.B. einer reibemittelhaltigen kosmetischen Formulierung zu erhöhen. Solche Thixotropiermittel sind beispielsweise für eine Vielzahl von Anwendungen als "rheologische Additive" bekannt, insbesondere zur Herstellung von Lacken und Anstrichstoffen, wobei sie als sogenanntes "Antiabsetzmittel" wirken. Hierbei handelt es sich beispielsweise um Bentonite und Kaoline.

In kosmetischen Formulierungen sind solche Thixotropiermittel auch für die Ausbildung einer festen sogenannten "Kartenhaus-Gelstruktur" verantwortlich. Insbesondere gilt dies für kosmetische Formulierungen, die polare organische Medien enthalten. Eine solche "Kartenhausstruktur" bildet sich jedoch bei Haut- und Handreinigungsmitteln, die ethoxylierte Fettalkohole als Reinigungsverstärker enthalten, nicht aus bzw. diese ist so massiv gestört, dass die Stabilisierung einer solchen kosmetischen Formulierung nicht mehr gewährleistet ist.

Überraschender Weise wurde nun gefunden, dass solche Systeme stabilisiert werden können, wenn diese kosmetischen Formulierungen, insbesondere Haut- und Handreinigungsmittel neben einem Thixotropiermittel eine hydrophile, pyrogene Kieselsäure enthalten.

Bei hydrophilen, pyrogenen Kieselsäuren handelt es sich um hochdisperse Kieselsäuren, d.h. um $SiO_2$-Modifikationen, die durch Flammenhydrolyse hergestellt worden sind, wobei die Oberflächen solcher pyrogenen Kieselsäuren einer chemischen Nachbehandlung unterzogen sein kann, so dass hierdurch das Spektrum der erhältlichen Kieselsäuren erweitert ist und auch Kieselsäuren mit hydrophobierten Oberflächen erhalten werden können. Die erfindungsgemäß einzusetzenden hydrophilen, pyrogenen Kieselsäuren der Komponente c.) sind beispielsweise unter dem Handelsnamen AEROSIL® erhältlich.

Die erfindungsgemäßen Haut- und Handreinigungsmittel enthalten > 0,1 Gew.-%, einer hydrophilen, pyrogenen Kieselsäure, insbesondere 0,5 bis 5,0 Gew.-%, bezogen auf die Gesamtzusammensetzung der Formulierung, wobei hydrophile, pyrogene Kieselsäuren, die unter dem Handelsnamen AEROSIL® 200 erhält- lich sind, besonders bevorzugt sind.

Thixotropiermittel der Komponente c.) sind zu 0,5 bis 10 Gew.-%, bezogen auf die Gesamtzusammensetzung, vorzugsweise 0,5 bis 7,5 und besonders bevorzugt 2 bis 6 Gew.-% in den erfindungsgemäßen Haut- und Handreinigungsmittel enthalten. Die Thixotropiermittel sind organophile und/oder hydrophobe Schichtsilikate, insbesondere Bentonite, vorzugsweise in denen die anorganischen Kationen natürlicher Bentonite, z.B. Na-Betonit durch organische Reste insbesondere quaternäre Ammoniumkationen ersetzt sind, wie beispielsweise bei den Bentoniten, die von der Fa. Rockwood Clay Additives, Moosburg, Deutschland unter dem Handelsnamen TIXOGEL® vertrieben werden.

Solche organischen Betonite können beispielsweise über die Bestimmung ihres Glühverlustes bei 1000°C charakterisiert werden, der als aussagefähiger Parameter für das Verhältnis von organischem Rest/Bentonite-Verhältnis angesehen werden kann. Der Glühverlust umfasst hierbei den gesamten organischen Anteil des Bentonites sowie das chemisch gebundene Wasser des Bentonits (ca. 8% bei reinem Bentonit).

Es wurde nun gefunden, dass Bentonite, die einen Glühverlust von max. 30% aufwiesen in Kombination mit einer hydrophilen, pyrogenen Kieselsäure in einem wasserfreien, lösemittelbasierten Haut- und Handreinigungsmitteln eine ausreichende Stabilität gewährleisten, sodass Produktinstabilitäten nicht mehr zu erkennen waren.

Erfindungsgemäß besonders bevorzugt sind hierbei gemäß INCI-Nomenklatur Stearalkoniumbentonite mit einem Glühverlust von max. 29% wie z. B. Stearalkoniumbentonite, die unter dem Handelsnamen TIXOGEL® LG-M von der Fa. Rockwood Clay Additives GmbH, Moosburg, Deutschland bezogen werden können und einen Glühverlust von ca. 28% aufweisen. Solche Stearalkoniumbentonite sind bevorzugt in einer Menge von > 0,5 Gew.-%, bezogen auf die Gesamtzusammensetzung und besonders bevorzugt > 2 Gew.-% in den erfindungsgemäßen Haut- und Handreinigungsmittel enthalten.

Es war überraschend, dass solche Stearalkoniumbentonite, die einen Glühverlust von max. 29% wie z. B. die unter dem Handelsnamen TIXOGEL® LG-M erhältlichen Stearalkoniumbentonite aufweisen, und bis- her insbesondere bei Nagellacken zur Stabilisierung von Farbpigmenten zur Anwendung gekommen sind, nunmehr auch zur Stabilisierung von wasserfreien bzw. wasserarmen kosmetischen Formulierungen, insbesondere für Haut- und Handreinigungsmittel eingesetzt werden können.

Die erfindungsgemäßen Haut- und Handreinigungsmittel zeigen eine sehr gute Reinigungswirkung und enthalten 5 bis 30 Gew.-% eines oder mehrerer Abrasiva, bezogen auf die Zusammensetzung des Reinigungsmittels, bevorzugt 5 bis 25 Gew.-%. Bevorzugte Abrasiva sind Kunststoffreibemittel auf der Basis von Polyethylen oder Polyurethan, Reibemittel auf der Basis von natürlichen Kern-, Hülsen- und/oder Schalenmehlen, insbesondere Walnußschalen-, Mandelschalen-, Haselnußschalen-, Olivenkern-, Aprikosenkern- und Kirschkern- und Maismehl oder beliebige Gemische dieser Schalen- und Kernmehle und Perlen aus Wachsen, wie z.B. Jojobawachse, wobei mit Wasserstoffperoxid gebleichtes Walnußschalenmehl besonders bevorzugt ist.

In einer Ausführungsform der Erfindung enthalten die erfindungsgemäßen Haut- und Handreinigungsmittel als Abrasiva wasserquellbare Feststoffteilchen von organischen Polymeren natürlichen und/oder synthetischen Ursprungs, die neben ihrer Wirkung als Reinigungsverstärker eine Koagulierung und Wiederablagerung des gelösten bzw. emulgierten Schmutzes bei Anwendung der Mittel verhindern.

Solche in Wasser unlöslichen, vielmehr lediglich wasserquellbaren Feststoffteilchen von organischen Polymeren natürlichen und/oder synthetischen Ursprungs als Abrasiva für Haut- und Handreinigungsmittel sind beispielsweise in der DE 37 36 970 beschrieben. Hierbei handelt es sich um Polymerisate auf Basis von modifizierten Naturstoffen oder auf Basis von synthetischen Produkten, wobei deren Wasserunlöslichkeit im Allgemeinen durch Vernetzung erzielt wird. Der Begriff Polymerisate umfasst dabei sowohl die Homoals auch die Co- und Terpolymeren.

Geeignete organische Polymere auf Basis modifizierter Naturstoffe sind beispielsweise die Produkte auf Stärke- und Cellulosebasis, die durch Pfropfung vorzugsweise mit Acrylderivaten modifiziert sein können. Solche Acrylderivate sind beispielsweise (Meth)acrylsäure und deren Salze, (Meth)acrylnitril, (Meth)acrylamid und die (Meth)acrylester, sowie die partiellen Verseifungsprodukte dieser Acrylderivate.

Als synthetische organische Polymere sind die Homo- und Copolymerisate, insbesondere der vorstehend genannten Acrylderivate zu nennen. Hierbei handelt es sich im wesentlichen um vernetzte Polyacrylsäuren oder vernetzte Stärke/Acrylsäure-Pfropfcopolymerisate, bei denen die Carboxylgruppen teilweise mit Natrium- oder Kaliumionen neutralisiert sein können. Als Comonomeren können die Polymerisate Acrylamidopropansulfonsäure, Vinylphosphonsäure, Vinylsulfonsäure, Dialkylaminoalkyl(meth-)acrylate, Dialkylamino(meth)-acrylamide sowie die quaternierten Formen der beiden vorstehend genannten basischen Comonomeren enthalten. Weiterhin sind auch Polyurethane geeignet.

Erfindungsgemäß bevorzugt sind Abrasiva in Form von wasserquellbaren Polymerisate, die durch Polymerisation der Komponenten

aa.) 55 bis 99,95 Gew.-% monoethylenisch ungesättigte Carboxylgruppen tragenden Monomeren,
bb.) 0,05 bis 5,0 Gew.-% wenigstens eines Vernetzungsmittels,
cc.) 0 bis 40 Gew.-% weiteren, mit a.) copolymerisierbaren Monomeren,
dd.) 0 bis 30 Gew.-% einer wasserlöslichen Pfropfgrundlage,

und sich die Bestandteile aa.) bis dd.) zu 100 Gew.-% ergänzen, erhältlich sind, wobei die erhaltenen Polymerisate gegebenenfalls mindestens einmal nachvernetzt sein können.

Als monoethylenisch ungesättigte Carboxylgruppen tragende Monomeren der Komponente aa.) sind hierbei insbesondere monoethylenisch ungesättigte $C_3$ bis $C_{10}$-Monocarbonsäuren sowie deren Alkali- und/oder Ammonium- und/oder Aminsalze zu nennen. Zu diesen Monomeren gehören beispielsweise Acrylsäure, Methacrylsäure, Dimethacrylsäure, Ethylacrylsäure, Crotonsäure, Isocrotonsäure, Vinylessigsäure und Allylessigsäure. Aus dieser Gruppe verwendet man Acrylsäure, Methacrylsäure bzw. deren Alkali- oder Ammoniumsalze oder deren Mischungen als bevorzugte Monomeren, wobei Acrylsäure sowie deren Natrium-, Kalium- oder Ammoniumsalze als Monomeren besonders bevorzugt sind.

Weitere monoethylenisch ungesättigte Carboxylgruppen tragende Monomeren sind auch die monoethylenisch ungesättigten $C_4$ bis $C_8$-Dicarbonsäuren, deren Anhydride bzw. deren Alkali- und/oder Ammonium- und/oder Aminsalze. Geeignete Dicarbonsäuren sind beispielsweise Maleinsäure, Fumarsäure, Itaconsäure und Methylenmalonsäure, wobei Maleinsäure, Maleinsäureanhydrid, Itaconsäure, Itaconsäureanhydrid sowie die entsprechenden Natrium-, Kalium- oder Ammoniumsalze von Malein- bzw. Itaconsäure bevorzugt sind.

Monoethylenisch ungesättigte Carboxylgruppen tragende Monomeren sind auch die Hydrolysate von (Meth)acrylnitrilcopolymeren und von Stärke-(Meth)acrylnitril-Pfropfcopolymeren, Hydrolysate von (Meth)acrylamidcopolymeren sowie Verseifungsprodukte von (Meth)acrylsäurecopolymeren mit ethylenisch ungesättigten Estern als Carboxylatgruppen enthaltendes Polymer.

Die sauren, einpolymerisierten Monomerbestandteile der wasserquellbaren Polymerisate sind vorzugsweise mindestens zu 25 Mol.% und bevorzugt zu mindestens 50 Mol.% und besonders bevorzugt zu mindestens 75 Mol.% neutralisiert und liegen, wie zuvor beschrieben, beispielsweise als Natrium-, Kalium-, oder Ammoniumsalz bzw. deren Gemische vor.

Als Vernetzungsmittel der Komponente bb.) werden üblicherweise Verbindungen eingesetzt, die mindestens zwei ethylenisch ungesättigte Doppelbindungen oder eine ethylenisch ungesättigte Doppelbindung und eine gegenüber Säuregruppen reaktive funktionelle Gruppe oder mehrere gegenüber Säuregruppen reaktive funktionelle Gruppen besitzen. Bevorzugte Vernetzungsmittel sind solche, die mindestens zwei ethylenisch ungesättigte Doppelbindungen enthalten, wie z.B. Methylenbisacrylamid bzw. methacrylamid oder Ethylenbisacrylamid, ferner Ester der ungesättigten Mono- oder Polycarbonsäuren von Polyolen, wie Diacrylate oder Triacrylate, z. B. Butandiol- oder Ethylenglykoldiacrylat bzw. -methacrylat, Trimethylolpropantriacrylat, sowie deren Alkoxylate mit vorzugsweise 1 bis 30 Mol Ethylenoxid, ferner Allylverbindungen und deren Alkoxylate wie Allyl(meth)acrylat, Allyl(EO)$_{1-30}$(meth)acrylat, Triallylcyanurat, Maleinsäurediallylester, Polyallylester, Tetraallyloxiethan, Di- und Triallylamin, Tetraallylethylendiamin, Allylester der Phosphorsäure bzw. phosphorigen Säure.

Verbindungen, die mindestens eine gegenüber Säuregruppen reaktive funktionelle Gruppe besitzen, sind beispielsweise die N-Methylolverbindungen von Amiden, wie z.B. Methacrylamid oder Acrylamid und die davon abgeleiteten Ether, aber auch Di- und Polyglycidylverbindungen sind hier zu nennen.

Die Vernetzungsmittel können alleine oder in Kombination in Mengen von 0,05 bis 5,0 Gew.-%, bevorzugt zu 0,05 bis 2,0 Gew.-% und besonders bevorzugt zu 0,1 bis 1,0 Gew.-%, bezogen auf die Monomeren, eingesetzt werden.

Optional können bei der Herstellung dieser wasserquellbaren Polymerisaten neben den monoethylenisch ungesättigte Carboxylgruppen tragenden Monomeren sowie dem Vernetzungsmittel (Komponenten aa.) und bb.)) weitere, in einer wässrigen Monomerlösung weitgehend lösliche Comonomere zur Modifizierung der Eigenschaften als Komponente cc.) enthalten sein. Solche Comonomere können beispielsweise (Meth)acrylamid, (Meth)acrylnitril, Vinylpyrrolidon, Vinylacetamid, 2-Acrylamido-2-methylpropansulfonsäure, Vinylsulfonsäure, (Meth)allylsulfonsäure, Hydroxyethylacrylat, Alkylpolyethylenglykol(meth)acrylate, Alkylaminoalkyl(meth)acrylate, Alkylaminopropylacrylamide, Acrylamidopropyltrimethylammoniumchlorid oder deren Gemische sein. Derartige Comonomere sollten einen Anteil von 40 Gew.-% nicht überschreiten, da sie die Quellfähigkeit des resultierenden wasserquellbaren Polymerisates gegebenenfalls beeinträchtigen können.

Darüber hinaus können die wasserquellbaren Polymerisate wasserlösliche Polymere als Komponente dd.) als Pfropfgrundlage enthalten, die optional in Mengen bis zu 30 Gew.-% enthalten sind. Dazu zählen unter anderem teil- oder vollverseifte Polyvinylalkohole, Polyacrylsäuren, Polyglykole oder deren Gemische, Polysaccharide wie z.B. Stärke oder Stärkederivate, Cellulose oder Cellulosederivate aber auch Polycarboxypolysaccharide. Letztere leiten sich entweder von Polysacchariden ab, die von Natur aus keine Carboxylgruppen enthalten, und durch nachträgliche Modifizierung mit Carboxylgruppen versehen werden oder sie enthalten von Natur aus bereits Carboxylgruppen und werden gegebenenfalls nachträglich durch Modifizierung mit weiteren Carboxylgruppen versehen.

Zur ersten Gruppe von Polysacchariden zählen beispielsweise Stärke, Amylose, Amylopektin, Cellulose und Polygalaktomannane wie Guar und Johannesbrotkernmehl, zur zweiten Gruppe zählen z.B. Xanthane, Alginate, Gummi Arabicum etc..

Die Carboxylgruppen sind, wie bereits erwähnt, entweder durch den von Natur aus gegebenen Molekülaufbau vorhanden, beispielsweise durch Uronsäureeinheiten im Polysaccharidmolekül oder werden durch nachträgliche Modifizierung mit carboxylgruppenhaltigen Reagenzien eingebaut bzw. durch Oxidationsreaktionen erzeugt. Unter den Polycarboxypolysacchariden, bei denen die Carboxylgruppen durch nachträgliche Modifizierung eingebaut werden, werden Carboxylalkylderivate bevorzugt, insbesondere die Carboxymethylderivate. Unter den Polycarboxypolysacchariden, bei denen die Carboxylgruppen durch Oxidation des Polysaccharidmoleküls erzeugt werden, werden insbesondere oxidierte Stärken und deren Derivate bevorzugt.

Polycarboxypolysaccharide können neben den Carboxylgruppen mit weiteren Gruppen modifiziert sein, insbesondere solchen, die die Wasserlöslichkeit verbessern, beispielsweise Hydroxyalkyl-, insbesondere Hydroxyethylgruppen, sowie Phosphatgruppen.

Besonders bevorzugte Polycarboxypolysaccharide sind Carboxymethylguar, carboxylierte Hydroxyethyl- oder Hydroxypropylcellulose, Carboxymethylcellulose und Carboxymethylstärke, oxidierte Stärke, carboxylierte Phosphatstärke, Xanthan und Mischungen aus den einzelnen Polycarboxypolysacchariden. Insbesondere wird Carboxymethylcellulose bevorzugt eingesetzt.

Polycarboxypolysaccharidderivate mit niedrigen und hohen Carboxyl-Substitutionsgraden sind einsetzbar. Üblicherweise weisen sie jedoch einen durchschnittlichen Carboxyl-Substitutionsgrad im Bereich von 0,3 bis 1,5 auf, wobei Polycarboxypolysaccharidderivate mit einem Substitutionsgrad im Bereich von 0,4 bis 1,2 bevorzugt eingesetzt werden.

Hinsichtlich der Komponente dd.) ist noch anzumerken, dass die Molekulargewichte der als Pfropfgrundlage zugesetzten Polymeren an die Gegebenheiten der Polymerisationsbedingungen angepasst sein müssen. So kann es z.B. im Falle einer wässrigen Lösungspolymerisation erforderlich sein, nur niedrig- oder mittelmolekulare Polymere einzusetzen, während dieser Faktor bei der Suspensionspolymerisation lediglich eine untergeordnete Rolle spielt.

Weiterhin ist bekannt, dass wasserquellbare Polymerisate durch das Verfahren der nachträglichen Oberflächenvernetzung in ihrem Eigenschaftsprofil verbessert werden können. Während einer solchen Nachvernetzung werden die Carboxylgruppen der Polymermoleküle an der Oberfläche der wasserquellbaren Polymerisatteilchen mit Vernetzungsmitteln bei erhöhter Temperatur vernetzt. Als Nachvernetzungsmittel werden Verbindungen eingesetzt, die mindestens zwei funktionelle Gruppen besitzen und die die funktionellen Gruppen des Polymerisates an der Oberfläche der Polymerteilchen vernetzen können Dabei sind Alkohol-, Amin-, Aldehyd-, Glycidyl-, Epichlor- und Isocyanatfunktionen bevorzugt, wobei auch Vernetzermoleküle mit mehreren verschiedenen Funktionen, aber auch mehrwertige Metallsalzverbindungen einsetzbar sind. Typische Beispiele für Nachvernetzungsmittel sind: Ethylenglykol, Diethylenglykol, Triethylenglykol, Glycerin, Polyglycerin, Propylenglykol, Diethanolamin, Triethanolamin, Sorbitanfettsäureester, Trimethylolpropan, Pentaerythrit, Polyvinylalkol, Sorbit, Ethylencarbonat, Proypylencarbonat, Polyepoxide wie z.B. Ethylenglykoldiglycidylether, Aziridine und Polyisocyanate. Bevorzugt wird mit Ethylencarbonat als Nachvernetzungsmittel gearbeitet. Die Nachvernetzungsmittel werden in einer Menge von 0,01 bis 10 Gew.-%, bevorzugt 0,1 bis 5 Gew.-%, besonders bevorzugt 0,1 bis 1,5 Gew.- % bezogen auf das nachzuvernetzende Polymerisat eingesetzt, wobei gegebenenfalls die nachträgliche Oberflächenvernetzung mehrfach wiederholt werden kann.

Bei der Herstellung der erfindungsgemäß zu verwendenden, Carboxylatgruppen enthaltenden wasserquellbaren Polymerisate können als Carboxylgruppen tragende Monomere beispielsweise Acrylsäure, Methacrylsäure, Vinylessigsäure, Maleinsäure bzw. deren Gemische eingesetzt werden. Die Verwendung von Acrylsäure allein bzw. deren Gemische ist bevorzugt.

Neben Polymerisaten, die durch vernetzende Polymerisation teilneutralisierter Acrylsäure erhalten werden, können auch solche verwendet werden, die zusätzliche Anteile von pfropfpolymerisierter Stärke und/oder Polyvinylalkohol enthalten.

Solche als Abrasiva wirkenden wasserquellbaren Feststoffteilchen können allein oder mit einem oder mehreren der oben genannten kosmetischen Abrasiva von 5 bis 30 Gew.-%, bezogen auf die Gesamtzusammensetzung des Haut- und Handreinigungsmittel, in den erfindungsgemäßen Haut- und Handreinigungsmittel enthalten sein.

Erfindungsgemäß bevorzugt sind wasserquellbare Abrasiva, die von der Firma Evonik Stockhausen, Krefeld (Deutschland) unter dem Handelsnamen Favor® T 5056 F erhältlich sind. Besonders bevorzugt sind als Komponente d) als Abrasiva wirkende wasserquellbare Polymerisate sowie gebleichtes Walnusschalenmehl, dass ebenfalls von der Firma Evonik Stockhausen unter der Bezeichnung ASTOPON® bezogen werden kann.

Weiterhin können die erfindungsgemäßen Haut- und Handreinigungsmittel optional als Komponente e.) 0 bis 5 Gew.-%, vorzugsweise 0,5 bis 2,5 Gew.-% mindestens einen physiologisch verträglichen Kohlensäureester, d.h. einen Kohlensäureester, der für kosmetische Anwendungen akzeptabel ist, vorzugsweise Propylencarbonat aufweisen.

Im Falle von wasserfreien Haut- und Handreinigungsmittelformulierungen bzw. der Verwendung von Stearalkoniumbentonite mit einem Glühverlust von max. 30% als Thixotropiermittel wirken solche Kohlensäurester wie z.B. Propylencarbonat nicht nur als Aktivierungsmittel, sondern bewirken in den erfindungsgemäßen Formulierungen neben einer Stabilisierung der Rezeptur, insbesondere bei Raumtemperatur (20°C) und bei der Kältelagerung (4°C) auch eine Antiabsetzwirkung, die durch andere Stearalkoniumbentonite mit einem Glühverlust von > 30% nicht erreicht werden konnte.

Die erfindungsgemäßen Haut- und Handreinigungsmittel sind vorzugsweise wasserfrei oder wasserarm bzw. enthalten lediglich 0 bis < 10 Gew.-% als Komponente f.).

Darüber hinaus können die erfindungsgemäßen Haut- und Handreinigungsmittel gegebenenfalls eines oder mehrere viskositätsbildende Mittel als Komponente g.) wie beispielsweise Polysaccharide, wie z.B. Cellulose, Guarmehl und/oder Xanthane, modifizierte Polysaccharide, bevorzugt Celluloseether, Carboxyalkylcellulose und/oder Hydroxyalkylcellulosen, vorzugsweise Hydroxyethylcellulose und/oder anorganische Elektrolyte, vorzugsweise Natriumchlorid und/oder Magnesiumsulfat.

Weiterhin können die erfindungsgemäßen Haut- und Handreinigungsmittel gegebenenfalls weitere kosmetische Hilfs-, Zusatz- und/oder Wirkstoffe, beispielsweise pH-Regulatoren, Stabilisatoren, vorzugsweise Cetearylalkohol und/oder hydrierte Ricinusöle, wie z.B. Trihydroxystearin, Duftstoffe, Konservierungsmittel, bevorzugt organische Säuren und Antioxidantien, wie z.B. Vitamin E-Acetat als Komponente h.) enthalten. Vorzugsweise können auch ölige oder wässrige Pflegekomponenten wie z.B. Bisabolol, Aloe vera, Panthenol, Sodium PCA, Jojobaöl, Creatin etc. eingesetzt werden, um die Pflegewirkung zu unterstreichen. Gleiches gilt für den gegebenenfalls zusätzlichen Einsatz von Pflegekomponenten wie hydrophilen Emollients, wie z.B. Partialglyceride oder Öle, durch die eine deutlich gesteigerte Hautpflegewirkung beobachtet werden kann bzw. eine sehr gute Pflegeeffekte werden mit Polyglycerylpartialestern, wie sie in der DE 10 2007 022 693 beschrieben worden sind. Weiterhin können auch übliche kosmetische Über- oder Rückfettungsmittel wie z.B. Isooctylstearate eingesetzt werden, um die Hautaustrocknungseffekte, insbesondere durch die Verwendung der eingesetzten Lösemittel zu minimieren.

Besonders vorteilhaft ist gegebenenfalls die zusätzliche Verwendung von hydriertem Ricinusöl wie z.B. RHEOCIN® (Fa. Rockwood Clay Additives, Moosburg, Deutschland) oder auch anderen Wachsen als Stabilisierungsmittel, vorzugsweise 0,5 bis 5 Gew.-%, besonders bevorzugt 0,5 bis 3 Gew.-%, bezogen auf die Gesamtzusammensetzung des Reinigungsmittels, insbesondere um nicht nur eine Stabilisierung bei unterschiedlichen Lagertemperaturen zu erreichen, sondern auch zur Erzielung der notwendigen Wärmestabilität der kosmetischen Formulierungen, insbesondere bei 40°C.

In diesem Zusammenhang ist zu betonen, dass stabile und lagerfähige Haut- und Handreinigungsmittel auf Basis von Alkylestern mit einem Wassergehalt < 10 Gew.-%, bezogen auf die Gesamtzusammensetzung, lediglich dann erhalten werden können, wenn diese Mittel erfindungsgemäß eine synergistische Kombination von mindestens einem Thixotropiermittel mit mindestens einer hydrophilen, pyrogenen Kieselsäure als Komponente aufweisen. Kosmetische Formulierungen mit Stearalkoniumbentonit sowie hydriertem Ricinusöl konnten Gesamtsystem nicht ausreichend stabilisieren. Erst durch den erfindungsgemäßen Zusatz einer hydrophilen Kieselsäure wie z.B. AEROSIL 200 konnte eine ausreichende Stabilisierung, insbesondere bei Raumtemperatur realisiert werden.

Die Herstellung der erfindungsgemäßen Haut- und Handreinigungsmittel, insbesondere Grobhandreiniger erfolgt üblicherweise mittels bekannter Vorrichtungen im Batch- oder kontinuierlichen Verfahren, wobei die Haut- und Handreinigungsmittel vorzugsweise als cremige Mittel oder als fließfähige viskose Pasten erhalten werden. Geeignete Vorrichtungen sind temperierbare Kessel mit Rührwerk, kontinuierliche Mischer wie Extruder und Dispergatoren.

Die Verwendung der erfindungsgemäßen Haut- und Handreinigungsmittel kann beispielsweise dergestalt erfolgen, indem zunächst bevorzugt ohne Wasser das Reinigungsmittel auf der Haut verteilt und anschließend mit einem Tuch, vorzugsweise ein Einwegartikel aus Papier, Kunststoff oder Textilgewebe etc., ohne Wasser abgewischt wird. Eine Anwendung unter Zuhilfenahme von Wasser ist jedoch auch möglich. Dabei wird das Produkt zusammen mit der Verschmutzung abgespült.

Die erfindungsgemäßen Haut- und Handreinigungsmittel werden zur Entfernung von stark auf der Haut haftenden Grobverschmutzungen, wie beispielweise Fette, Öle und andere Schmierstoffe, Farben, Lacke, Teer, Graphit, Ruß, Farbpigmente und ähnliche Stoffe eingesetzt wie sie im industriellen und öffentlichen Bereich, im Handwerk, in der Landwirtschaft sowie auch im Haushalt vorkommen. Besonders vorteilhaft sind die erfindungsgemäßen Haut- und Handreinigungsmittel bei der Reinigung von hartnäckigsten Lackverschmutzungen, wobei hierbei das Reinigungsmittel mindestens 10 Gew.-%, auf die Zusammensetzung, Fett- alkoholethoxylate enthalten sollte.

Besonders bevorzugte Haut- und Handreinigungsmittel sind wasserfrei und enthalten mindestens 10 Gew.-% die entsprechenden Methylester der erfindungsgemäßen Alkylester und/oder Diester als Komponente a.) und mindestens 5 Gew.-% Fettalkoholethoxylat als Komponente b.) vorzugsweise Laureth-6.

Die Erfindung wird durch folgende Beispiele und Untersuchungen, wie Hautverträglichkeitsprüfung mit Hilfe des Duhring-Kammer-Testes, Hautaustrocknung mit Hilfe des Corneometers und Reinigungskraft mit Hilfe des Handwaschtests beschrieben.

**Prüfmethoden**

**Stabilitätsprüfung**

Die Stabilitätsprüfung wurde in Anlehnung an den IFSCC Monograph 1992 Number 2 "The Fundamentals of Stability Testing" durchgeführt. Dort werden im Kapitel IV Seite 8 Standard Test conditions beschrieben, die als Grundlage für die durchgeführten Stabilitätstests dienten. Es wurden die Standardtestbedingungen im Kapitel IV.i.a. bei 4°C, RT und 40°C über einen zeitraum von 3 monate verwerdet.

**Prüfung der Reinigungskraft mit Hilfe des Handwaschtests**

Das Testmodell des Handwaschtests mit Lack gibt Auskunft über die Reinigungswirkung der zu prüfenden Produkte. Für eine Praxisrelevanz ist es erforderlich, dass alle Probanden eine charakteristische, durch manuelle Arbeit bedingte Hautstruktur der Handinnenflächen besitzen. Morgens und nachmittags wird mit je einem Produkt folgender Test durchgeführt:

**Testdurchführung mit Wasser:**

- 0,5 g Schmutz (Lack) werden auf die Handinnenfläche und auf den Handrücken verteilt und verrieben
- 1 ½ min. trocknen lassen
- 1,2 g Reinigungsmittel werden aufgetragen und eingerieben
- 1 ml Wasser wird zugefügt und 30 s gewaschen
- nochmals 1 ml Wasser zufügen und 30 s waschen lassen
- abspülen unter fließendem kalten Wasser
- visuelle Beurteilung der Restverschmutzung (RV) auf dem Handrücken und der Handinnenfläche gemäß Skala s.u.

0 = sauber 5 = kein Reinigungseffekt (Abstufung in 0,5er Schritten möglich)
Die Berechnung des prozentualen Reinigungseffektes erfolgt nach folgender Formel:

$$\text{Reinigungseffekt [\%]} = \frac{10 - (\overline{RV}_{innen} + \overline{RV}_{außen})}{10} * 100\%$$

$\overline{RV}_{innen}$ = Mittelwert der Restverschmutzung Handinnenflächen von n Meßreihen (Probanden)

$\overline{RV}_{außen}$ = Mittelwert der Restverschmutzung Handaußenflächen von n Meßreihen (Probanden)

Da die Bestimmung der Reinigungswirkung infolge der Testmethode eine höhere Schwankungsbreite aufweist, ist eine Absolutabweichung von 5% zwischen zwei Meßreihen zulässig.
Eine gute Reinigungswirkung ist erzelt, wenn der Reinigungseffekt bei min. 90% liegt. Bei niedrigeren prozentualen werten erkennt man bereits deutliene Restanschmutzungen bei der Händereiniguang.

Eingesetzter Lack:

Zero Glanzcolor Buntlack

**Ausführungsbeispiele:**

Es wurden Haut- und Handreinigungsmittel gemäß den in den Tabellen 1 und 2 angegebenen Zusammensetzungen durch homogene Dispergierung aller Komponenten bei Raumtemperatur hergestellt, wobei sicherzustellen ist, dass das unter der Bezeichnung RHEOCIN® von der Fa. Rockwood Clay Additives, Moos- burg, Deutschland erhältliche organische Geliermittel bei mindestens 40°C in die Formulierung eingearbeitet wird.

Die Mittel wurden im Hinblick auf ihre Reinigungswirkung gegenüber einem Lack und im Hinblick auf Ihre Stabilität charakterisiert.

Erfindungsgemäße Haut- und Handreinigungsmittel haben, wie in den nachfolgenden Tabellen 1, 2 und 3 dargestellt beispielsweise folgende Zusammensetzungen:

Tabelle 1:

| Alle Rezepturbeispiele in Gew.-% | 1 | 2 | 3 | 4 | 5 | 6 | 7 | 8 | 9 | 10 |
|---|---|---|---|---|---|---|---|---|---|---|
| Fettsäurealkylester ab C6, insbesondere Pflanzenölester wie z.B. Rapsmethylester oder Soyafettsäuremethylester | 54,60 | 50,50 | 50,50 | 50,50 | 50,50 | 64,60 | 11,00 | 40,00 | 48,00 | 47,50 |
| Fettalkoholethoxylat (z.B. Rewopal LA6) | 15,00 | 19,50 | 19,50 | 19,50 | 19,50 | 10,00 | 5,00 | 30,00 | 20,00 | 20,00 |
| Stearalkoniumbentonit (z.B.TIXOGEL LG-M) | 4,00 | 4,00 | 4,00 | 5,00 | 5,00 | 3,50 | 3,00 | 5,00 | 4,00 | 4,00 |
| Kieselsäure hydrophil (z.B.AEROSIL 200) | 2,50 | 2,50 | 2,50 | 1,50 | 1,00 | 1,00 | 2,00 | 1,50 | 2,00 | 1,50 |
| RHEOCIN® Stabilisator | 1,00 | 1,50 | 1,00 | 1,50 | 2,00 | 1,00 | 1,00 | 0,50 | - | - |
| Polyvinylpyrrolidon | - | - | - | - | - | - | - | - | 1,00 | 2,00 |
| Favor® T 5056 F | 4,00 | 4,00 | 4,00 | 4,00 | 4,00 | 4,00 | 4,00 | 4,00 | 4,00 | 4,00 |
| Alkylencarbonat Propylencarbonat | 1,00 | 1,00 | 1,00 | 1,00 | 1,00 | 1,00 | 1,00 | 1,00 | 1,00 | 1,00 |
| Walnussschalenmehl (Astopon® fein) | 13,00 | 12,10 | 12,60 | 12,10 | 12,10 | 10,00 | 20,00 | 13,00 | 15,00 | 15,00 |
| Calciumcarbonat | - | - | - | - | - | - | 20,00 | | | |
| Kaolin | - | - | - | - | - | - | 27,00 | - | | |
| Titandioxid | 1,0 | 1,0 | 1,0 | 1,0 | 1,0 | 1,0 | 1,0 | 1,0 | 1,0 | 1,0 |
| Isooctylstearat Überfettungsmittel | 3,90 | 3,90 | 3,90 | 3,90 | 3,90 | 3,90 | 5,00 | 4,00 | 4,00 | 4,00 |
| Gesamt | 100,00 | 100,00 | 100,00 | 100,00 | 100,00 | 100,00 | 100,00 | 100,00 | 100.00 | 100,00 |
| Stabilität bei -4°/20°C/40°C | stabil | stabil | stabil | stabil | stabil | stabil | stabil | stabil | stabil | stabil |
| | 98 | 97 | 98 | 98 | 97 | 95 | 92 | 98 | 95 | 95 |

Tabelle 2:

| Alle Rezepturbeispiele in Gew.-% | 11 | 12 | 13 | 14 | 15 | 16 | 17 | 18 | 19 |
|---|---|---|---|---|---|---|---|---|---|
| Carbonsäureester vom Typ Dimethyladipat, Dimethylglutarat, Dimethylsuccinat (DBE) | 54,60 | 50,50 | 50,50 | 50,50 | 50,50 | 64,60 | 48,00 | - | - |
| Diisobutyladipat/Diisobutylglutarat /Diisobutylsuccinat (Rhodiasolv DIB) | - | - | - | - | - | - | - | - | 55,00 |
| (Dimethyl 2-methyl glutarat/Dimethyl ethylsuccinat/Dimethyladipat) Rhodiasolv IRIS | - | - | - | - | - | - | - | 55,00 | - |
| Fettalkoholethoxylat (z.B. Rewopal LA6) | 15,00 | 19,50 | 19,50 | 19,50 | 19,50 | 10,00 | 20,00 | 15,00 | 15,00 |
| Stearalkoniumbentonit (z.B.TIXOGEL LG-M) | 4,00 | 4,00 | 4,00 | 5,00 | 5,00 | 3,50 | 4,00 | 4,00 | 4,00 |
| Kieselsäure hydrophil (z.B.AEROSIL 200) | 2,50 | 2,50 | 2,50 | 1,50 | 1,00 | 1,00 | 2,00 | 1,50 | 1,50 |
| RHEOCIN® Stabilisator | 1,00 | 1,50 | 1,00 | 1,50 | 2,00 | 1,00 | - | 1,50 | 1,50 |
| Polyvinylpyrrolidon | - | - | - | - | - | - | 1,00 | | |
| Favor® T 5056 F | 4,00 | 4,00 | 4,00 | 4,00 | 4,00 | 4,00 | 4,00 | 4,00 | 4,00 |
| Alkylencarbonat Propylencarbonat | 1,00 | 1,00 | 1,00 | 1,00 | 1,00 | 1,00 | 1,00 | 1,00 | 1,00 |
| Walnussschalenmehl (Astopon® fein) | 13,00 | 12,10 | 12,60 | 12,10 | 12,10 | 10,00 | 15,00 | 13,00 | 13,00 |
| Calciumcarbonat | - | - | - | - | - | - | - | - | - |
| Kaolin | - | - | - | - | - | - | - | - | - |
| Titandioxid | 1,0 | 1,0 | 1,0 | 1,0 | 1,0 | 1,0 | 1,0 | 1,0 | 1,0 |
| Isooctylstearat Überfettungsmittel | 3,90 | 3,90 | 3,90 | 3,90 | 3,90 | 3,90 | 4,00 | 4,00 | 4,00 |
| Gesamt | 100,00 | 100,00 | 100,00 | 100,00 | 100,00 | 100,00 | 100,00 | 100,00 | 100,00 |
| Stabilität bei -4°/20°C/40°C | stabil | stabil | stabil | stabil | stabil | stabil | stabil | stabil | stabil |

Rhodiasolv DIB (Diisobutyladipat/ Diisobutylglutarat/Diisobutylsuccinat) sowie Rhodiasolv IRIS (Dimetyl 2-methyt glutarat/ Dimethyl ethylsuccinat/ Dimethyladipat) sind Handelsprodukte der Fa. Rhodia France, 93306 Aubervilliers Cedex.

Tabelle 3:

| Alle Rezepturbeispiele in Gew.-% | 20 | 21 | 22 | 23 | 24 | 25 | 26 | 27 | 28 | 29 |
|---|---|---|---|---|---|---|---|---|---|---|
| Wasser | 0 | 0 | 0 | 9,50 | 10,50 | 12,50 | 15,00 | 30,00 | 15,00 | 20,00 |
| Metyloleate | 52,10 | 52,10 | 52,10 | 45,50 | 44,50 | 42,50 | 40,00 | 24,90 | 34,90 | 36,20 |
| BHT | | | | 0,1 | 0,10 | 0,10 | 0,10 | 0,10 | 0,10 | 0,10 |
| Fettalkoholethoxylat (z.B. Marlipal O13/60) | 15,00 | 15,00 | 15,00 | 15,00 | 15,00 | 15,00 | 15,00 | 15,00 | 20,00 | 10,00 |
| Fettalkoholethersulfat 28%ig | 0 | 0 | 0 | 0 | 0 | 0 | 0 | 0 | 0 | 10,00 |
| Stearalkoniumbentonit (z.B.TIXOGEL LG-M) | 3,50 | 3,50 | 3,50 | 3,50 | 3,50 | 3,50 | 3,50 | 3,50 | 3,50 | 3,50 |
| Kieselsäure hydrophil (z.B.AEROSIL 200) | 2,50 | 2,50 | 2,50 | 2,50 | 2,50 | 2,50 | 2,50 | 2,50 | 2,50 | 2,50 |
| RHEOCIN® Stabilisator | 1,00 | 1,00 | 1,00 | 1,00 | 1,00 | 1,00 | 1,00 | 1,00 | 1,00 | 1,00 |
| CMC (Walocel CRT 2000) | 0 | 0 | 0 | 0 | 0 | 0 | 0 | 0 | 0 | 0,70 |
| Favor® T 5056 F | 4,00 | 4,00 | 4,00 | 4,00 | 4,00 | 4,00 | 4,00 | 4,00 | 4,00 | 0 |
| Alkylencarbonat z.B. Propylencarbonat | 1,00 | 1,00 | 1,00 | 1,00 | 1,00 | 1,00 | 1,00 | 1,00 | 1,00 | 0 |
| Walnussschalenmehl | 13,00 | 13,00 | 13,00 | 13,00 | 13,00 | 13,00 | 13,00 | 13,00 | 13,00 | 15,00 |
| Cocamide MEA | 3,00 | 0 | 0 | 0 | 0 | 0 | 0 | 0 | 0 | 0 |
| Cocamide MIPA | 0 | 3,00 | 0 | 0 | 0 | 0 | 0 | 0 | 0 | 0 |
| Cocamide DEA | 0 | 0 | 3,00 | 0 | 0 | 0 | 0 | 0 | 0 | 0 |
| Titandioxid | 1,0 | 1,0 | 1,0 | 1,0 | 1,0 | 1,0 | 1,0 | 1,0 | 1,0 | 1,0 |
| Isooctylstearat Überfettungsmittel | 3,90 | 3,90 | 3,90 | 3,90 | 3,90 | 3,90 | 3,90 | 4,0 | 4,00 | 0 |
| Gesamt | 100,00 | 100,00 | 100,00 | 100,00 | 100,00 | 100,00 | 100,00 | 100,00 | 100,00 | 100,00 |
| Stabilität bei -4°/20°C/40°C | stabil | stabil | stabil | stabil | instabil | instabil | instabil | instabil | instabil | instabil |
| Reinigungseffekt in % | 98 | 98 | 98 | 90 | 84 | 76 | 66 | 50 | 69 | 63 |
| BHT steht für Butylhydroxytoluol (2,6-Di-tert-butyl-4-methylphenol) | | | | | | | | | | |

Methyoloeate ist eine Mischung aus $C_{16}$- $C_{18}$ Methylester, worin der $C_{18}$-Methylester enine Anteil von > 50 Gew.-% hat.

Die Rezepturbeispiele 24 bis 29 sind nicht erfindungsgemäße Vergleichsbeispiele. Diese waren instabil und reinigten significant schleter, als die erfindungsgemäße Beispiele 1 bis 23.

## Patentansprüche

1. Haut- und Handreinigungsmittel enthaltend die Komponenten

   a.) mindestens einen Alkylester und/oder Diester,
   b.) 1 bis 40 Gew.-% mindestens eines Tensids ausgewählt aus der Gruppe der Fettalkoholethoxylate, Fettalkoholethersulfate und Salze sulfierter und/oder sulfonierter Fettsäuren,
   c.) 0,5 bis 10 Gew.-%, bezogen auf die Gesamtzusammensetzung, mindestens eines Thixotropiermittels, ausgewähit aus organophilen und/oder hydrophoben Schichtsilikaten, und > 0,1 Gew.-%, bezogen auf die Gesamtzusammensetzung, mindestens einer hydrophilen, pyrogenen Kieselsäure,
   d.) 5 bis 30 Gew.-% eines oder mehrerer Abrasiva ausgewähit aus Sand, Bimsmehl, Calciumcarbonat, Kaolin, Kunststoffreibemittel auf der Basis von Polyethylen oder Polyurethan, Reibemittel auf der Basis von natürlichen Kern-, Hülsen- und/oder Schalenmehlen, oder Gemische dieser Schalen- und Kernmehle und/oder Perlen aus Wachsen, und/oder wasserquellbare Feststoffteilchen von organischen Polymeren natürlichen und/oder synthetischen Ursprungs
   e.) 0 bis 5 Gew.-% mindestens einen physiologisch verträglichen Kohlensäureester
   f.) 0 bis < 10 Gew,-% Wasser,
   g.) gegebenenfalls eines oder mehrere viskositätsbildende Mittel,
   h.) gegebenenfalls weitere kosmetische Hilfs-, Zusatz- und/oder Wirkstoffe,

   wobei die Summe der Komponenten a.) bis h.) 100 Gew.-%, bezogen auf die Zusammensetzung des Reinigungsmittels, ergibt.

2. Haut- und Handreinigungsmittel nach Anspruch 1, **dadurch gekennzeichnet, dass** sie als Komponente a.) Fettsäurealkylester der allgemeinen Formel (I) aufweisen, nämlich

   $$R^1CO\text{-}OR^2 \qquad (I)$$

   in der $R^1CO$ für einen linearen oder verzweigten, gesättigten und/oder ungesättigten Acylrest mit 6 bis 22 und $R^2$ für einen Alkylrest mit 1 bis 8 Kohlenstoffatomen steht.

3. Haut- und Handreinigungsmittel nach Anspruch 1 oder 2, **dadurch gekennzeichnet, dass** sie als Komponente a.) Diester der allgemeinen Formel (ii)

   $$R^1OOC\text{-}X\text{-}COOR^2 \qquad (ii)$$

   aufweisen, in der X für einen linearen oder verzweigten, gesättigten und/oder ungesättigten Alkylrest mit 0 bis 20 und $R^1$ und $R^2$ für einen Alkylrest mit 1 bis 8 Kohlenstoffatomen steht.

4. Haut- und Handreinigungsmittel nach einem der Ansprüche 1 bis 3, **dadurch gekennzeichnet, dass** sie 5 bis 70 Gew.-%, bezogen auf die Gesamtzusammensetzung, Alkylester und/oder Diester der Komponente a.) enthalten.

5. Haut- und Handreinigungsmittel nach einem der Ansprüche 1 bis 4, **dadurch gekennzeichnet, dass** sie als Komponente b.) mindestens ein Fettalkoholethoxylat der allgemeinen Formel

   $$R\text{-}O\text{-}(CH_2\text{-}CH_2\text{-}O)_nH$$

   enthalten, wobei
   R = gesättigter, ungesättigter, verzweigter oder unverzweigter Alkylrest und n = ganze Zahl von 1 bis 11 sind.

6. Haut- und Handreinigungsmittel nach einem der Ansprüche 1 bis 5, **dadurch gekennzeichnet, dass** sie 1 bis 35 Gew.-%, bezogen auf die Gesamtzusammensetzung, mindestens eines Fettalkoholethoxylats als Komponente b.)

enthalten.

7. Haut- und Handreinigungsmittel nach den Ansprüchen 1 bis 6, **dadurch gekennzeichnet, dass** sie 5 bis 35 Gew,-%, bezogen auf die Gesamtzusammensetzung, Laureth-6 als Fettalkoholethoxylat der Komponente b.) aufweisen.

8. Haut- und Handreinigungsmittel nach den Ansprüchen 1 bis 7, **dadurch gekennzeichnet, dass** sie mindestens ein Fettalkoholethersulfat der allgemeinen Formel

$$R\text{-}O\text{-}(CH_2\text{-}CH_2\text{-}O)_n SO_3 X$$

enthalten,

mit R = einem $C_8$-$C_{18}$ gesättigten oder ungesättigten, verzweigten oder unverzweigten Alkylrest, n = einer ganzen Zahl von1 bis 6 und
X = $Na^+$, $NH_4^+$ oder $\frac{1}{2}Mg^{2+}$.

9. Haut- und Handreinigungsmittel nach den Ansprüchen 1 bis 8, **dadurch gekennzeichnet, dass** sie als Komponente c.) 0,5 bis 7,5 Gew.-%, bezogen auf die Gesamtzusammensetzung, mindestens ein Thixotropiermittel und 0,5 Gew.-% bis 5,0 Gew.-%, bezogen auf die Gesamtzusammensetzung, mindestens eine hydrophile, pyrogene Kieselsäure, enthalten.

10. Haut- und Handreinigungsmittel nach den Ansprüchen 1 bis 9, **dadurch gekennzeichnet, dass** das Thixotropiermittel der Komponente c.) ein hydrophober Bentonit ist.

11. Haut- und Handreinigungsmittel nach den Ansprüchen 1 bis 10, **dadurch gekennzeichnet, dass** das Thixotropiermittel der Komponente c.) ein Stearalkoniumbentonit ist.

12. Haut- und Handreinigungsmittel nach den Ansprüchen 1 bis 11, **dadurch gekennzeichnet, dass** es als Abrasiva der Komponente d.) Kunststoffreibemittel auf der Basis von Polyethylen oder Polyurethan, oder Reibemittel auf der Basis von natürlichen Kern-, Hülsen- und/oder Schalenmehlen, -ausgewählt aus Walnußschalen-, Mandelschalen-, Haselnußschalen-, Olivenkern-, Aprikosenkern-, Kirschkern- und Maismehl oder Gemische dieser Schalen- und Kernmehle aufweist.

13. Haut- und Handreinigungsmittel nach den Ansprüchen 1 bis 12, **dadurch gekennzeichnet, dass** die Abrasiva der Komponente d.) in Form wasserquellbarer Polymerisate durch Polymerisation der Komponenten

aa.) 55 bis 99,95 Gew.-% monoethylenisch ungesättigte Carboxylgruppen tragenden Monomeren,
bb.) 0,05 bis 5,0 Gew.-% wenigstens eines Vernetzungsmittels,
cc.) 0 bis 40 Gew.-% weiteren, mit a.) copolymerisierbaren Monomeren,
dd.) 0 bis 30 Gew.-% einer wasserlöslichen Pfropfgrundlage,

und sich die Bestandteile aa.) bis dd.) zu 100 Gew.-% ergänzen, erhältlich sind, wobei die erhaltenen Polymerisate gegebenenfalls mindestens einmal nachvernetzt sein können.

14. Haut- und Handreinigungsmittel nach den Ansprüchen 1 bis 13, **dadurch gekennzeichnet, dass** sie wasserfrei sind.

**Claims**

1. Skin and hand cleansers containing the components

a.) at least one alkyl ester and/or diester,
b.) from 1 to 40% by weight of at least one surfactant selected from the group of fatty alcohol ethoxylates, fatty alcohol ether sulphates and salts of sulphated and/or sulphonated fatty acids,
c.) from 0.5 to 10% by weight, with respect to the total composition, of at least one thixotropic agent, selected from organophilic and/or hydrophobic layer silicates, and > 0.1% by weight, with respect to the total composition, of at least one hydrophilic, pyrogenic silicic acid,
d.) from 5 to 30% by weight of one or more abrasives selected from sand, pumice powder, calcium carbonate, kaolin, synthetic abrasives based on polyethylene or polyurethane, abrasives based on natural stone, husk and/or shell flours, or admixtures of these shell and stone flours and/or pearls of wax, and/or water-swellable solid particles of organic polymers of natural and/or synthetic origin,
e.) from 0 to 5% by weight of at least one physiologically compatible carbonic acid ester,
f.) from 0 to <10% by weight of water,
g.) where applicable, one or more viscosity-forming agents,
h.) where applicable, additional cosmetic auxiliary agents, additives and/or active ingredients,

wherein the sum of the components a.) to h.) is 100% by weight with respect to the composition of the cleanser.

2. Skin and hand cleansers according to claim 1, **characterised in that** they have as component a.) fatty acid alkyl esters of the general formula (I), that is to say,

$$R^1CO\text{-}OR^2 \qquad (I)$$

where $R^1CO$ stands for a linear or branched, saturated and/or unsaturated acyl residue with from 6 to 22 carbon atoms and $R^2$ stands for an alkyl residue with from 1 to 8 carbon atoms.

3. Skin and hand cleansers according to claim 1 or claim 2, **characterised in that** they have as component a.) diesters of the general formula (ii)

$$R^1OOC\text{-}X\text{-}COOR^2 \qquad (ii)$$

in which X stands for a linear or branched, saturated and/or unsaturated alkyl residue with from 0 to 20 carbon atoms and $R^1$ and $R^2$ stand for an alkyl residue with from 1 to 8 carbon atoms.

4. Skin and hand cleansers according to any one of claims 1 to 3, **characterised in that** they contain from 5 to 70% by weight, with respect to the total composition, of alkyl esters and/or diesters of the component a.).

5. Skin and hand cleansers according to any one of claims 1 to 4, **characterised in that** they contain as component b.) at least one fatty alcohol ethoxylate of the general formula

$$R\text{-}O\text{-}(CH_2\text{-}CH_2\text{-}O)_nH$$

where
R = saturated, unsaturated, branched or non-branched alkyl residue and n = a whole number from 1 to 11.

6. Skin and hand cleansers according to any one of claims 1 to 5, **characterised in that** they contain from 1 to 35% by weight, with respect to the total composition, of at least one fatty alcohol ethoxylate as component b.)

7. Skin and hand cleansers according to claims 1 to 6, **characterised in that** they have from 5 to 35% by weight, with respect to the total composition, of laureth-6 as a fatty alcohol ethoxylate of the component b.).

8. Skin and hand cleansers according to claims 1 to 7, **characterised in that** they contain at least one fatty alcohol ether sulphate of the general formula

$$R\text{-}O\text{-}(CH_2\text{-}CH_2\text{-}O)_nSO_3X$$

with R = a $C_8$-$C_{18}$-saturated or unsaturated, branched or non-branched alkyl residue, n = a whole number from 1 to 6, and
X = $Na^+$, $NH_4^+$ or $\frac{1}{2}Mg^{2+}$.

9. Skin and hand cleansers according to claims 1 to 8, **characterised in that** they contain as component c.) from 0.5 to 7.5% by weight, with respect to the total composition, of at least one thixotropic agent and from 0.5% by weight to 5.0% by weight, with respect to the total composition, of at least one hydrophilic, pyrogenic silicic acid.

10. Skin and hand cleansers according to claims 1 to 9, **characterised in that** the thixotropic agent of the component c.) is a hydrophobic bentonite.

**EP 2 278 952 B1**

11. Skin and hand cleansers according to claims 1 to 10, **characterised in that** the thixotropic agent of the component c.) is a stearalkonium bentonite.

12. Skin and hand cleansers according to claims 1 to 11, **characterised in that** it has as abrasives of the component d.) synthetic abrasives based on polyethylene or polyurethane, or abrasives based on natural stone, husk and/or shell flours, selected from walnut shell flour, almond shell flour, hazelnut shell flour, olive stone flour, apricot stone flour, cherry stone flour and corn flour or admixtures of these shell and stone flours.

13. Skin and hand cleansers according to claims 1 to 12, **characterised in that** the abrasives of the component d.) in the form of water-swellable polymerisates can be obtained by means of polymerisation of the components

aa.) from 55 to 99.95% by weight of monomers carrying monoethylenically unsaturated carboxyl groups,
bb.) from 0.05 to 5.0% by weight of at least one wetting agent,
cc.) from 0 to 40% by weight of additional monomers which can be copolymerised with a.),
dd.) from 0 to 30% by weight of water-soluble clotting base,

and the components aa.) to dd.) total 100% by weight, wherein the polymerisates obtained can where applicable be post-cross-linked at least once.

14. Skin and hand cleansers according to claims 1 to 13, **characterised in that** they are water-free.


**Revendications**

1. Agent de nettoyage de la peau et des mains contenant les composants

a.) au moins un alkylester et/ou diester,
b.) 1 à 40 % en poids d'au moins un tensioactif choisi dans le groupe des produits d'éthoxylation d'alcools gras, des éthersulfates d'alcools gras et des sels d'acides gras sulfatés et/ou sulfonés,
c.) 0,5 à 10 % en poids, par rapport à la composition totale, d'au moins un agent de thixotropie, choisi parmi les silicates feuilletés organophiles et/ou hydrophobes, et > 0,1 % en poids, par rapport à la composition totale, d'au moins un acide silicique pyrogène hydrophile,
d.) 5 à 30 % en poids d'un ou plusieurs abrasifs choisis parmi le sable, la farine de pierre ponce, le carbonate de calcium, le kaolin, les agents de frottement en matière synthétique à base de polyéthylène ou de polyuréthane, les agents de frottement à base de farines de noyaux, d'enveloppes et/ou de coquilles naturelles, ou les mélanges de ces farines de coquilles et de noyaux et/ou les perles de cires, et/ou les particules solides gonflables à l'eau de polymères organiques d'origine naturelle et/ou synthétique
e.) 0 à 5 % en poids d'au moins un ester d'acide carbonique physiologiquement acceptable
f.) 0 à < 10 % en poids d'eau,
g.) éventuellement un ou plusieurs agents produisant de la viscosité,
h.) éventuellement d'autres adjuvants, additifs et/ou principes actifs cosmétiques,

où la somme des composants a.) à h.) donne 100 % en poids, par rapport à la composition de l'agent de nettoyage.

2. Agents de nettoyage de la peau et des mains selon la revendication 1, **caractérisés en ce qu'**ils présentent comme composant a.) des alkylesters d'acides gras de formule générale (I), à savoir

$$R^1CO\text{-}OR^2 \qquad (I)$$

dans laquelle $R^1CO$ représente un groupement acyle linéaire ou ramifié, saturé et/ou insaturé, avec 6 à 22 et $R^2$ représente un groupement alkyle avec 1 à 8 atomes de carbone.

3. Agents de nettoyage de la peau et des mains selon la revendication 1 ou 2, **caractérisés en ce qu'**ils présentent comme composant a.) des diesters de formule générale (ii)

$$R^1OOC\text{-}X\text{-}COOR^2 \qquad (ii)$$

dans laquelle X représente un groupement alkyle linéaire ou ramifié, saturé et/ou insaturé avec 0 à 20 et $R^1$ et $R^2$

représentent un groupement alkyle avec 1 à 8 atomes de carbone.

4. Agents de nettoyage de la peau et des mains selon l'une des revendications 1 à 3, **caractérisés en ce qu'**ils contiennent 5 à 70 % en poids, par rapport à la composition totale, d'alkylesters et/ou diesters du composant a.).

5. Agents de nettoyage de la peau et des mains selon l'une des revendications 1 à 4, **caractérisés en ce qu'**ils contiennent comme composant b.) au moins un produit d'éthoxylation d'alcool gras de formule générale

$$R\text{-}O\text{-}(CH_2\text{-}CH_2\text{-}O)_nH$$

où
R = groupement alkyle saturé, insaturé, ramifié ou non ramifié et n = nombre entier de 1 à 11.

6. Agents de nettoyage de la peau et des mains selon l'une des revendications 1 à 5, **caractérisés en ce qu'**ils contiennent 1 à 35 % en poids, par rapport à la composition totale, d'au moins un produit d'éthoxylation d'alcool gras comme composant b.).

7. Agents de nettoyage de la peau et des mains selon les revendications 1 à 6, **caractérisés en ce qu'**ils présentent 5 à 35 % en poids, par rapport à la composition totale, de laureth-6 comme produit d'éthoxylation d'alcool gras du composant b.).

8. Agents de nettoyage de la peau et des mains selon les revendications 1 à 7, **caractérisés en ce qu'**ils contiennent au moins un éthersulfate d'alcool gras de formule générale

$$R\text{-}O\text{-}(CH_2\text{-}CH_2\text{-}O)_nSO_3X$$

avec R = un groupement alkyle en $C_8$-$C_{18}$ saturé ou insaturé, ramifié ou non ramifié, n = un nombre entier de 1 à 6 et
X = $Na^+$, $NH_4^+$ ou $\frac{1}{2}$ $Mg^{2+}$.

9. Agents de nettoyage de la peau et des mains selon les revendications 1 à 8, **caractérisés en ce qu'**ils contiennent comme composant c.) 0,5 à 7,5 % en poids, par rapport à la composition totale, d'au moins un agent de thixotropie et 0,5 % en poids à 5,0 % en poids, par rapport à la composition totale, d'au moins un acide silicique hydrophile pyrogène.

10. Agents de nettoyage de la peau et des mains selon les revendications 1 à 9, **caractérisés en ce que** l'agent de thixotropie du composant c.) est une bentonite hydrophobe.

11. Agents de nettoyage de la peau et des mains selon les revendications 1 à 10, **caractérisés en ce que** l'agent de thixotropie du composant c.) est une bentonite de stéaralkonium.

12. Agent de nettoyage de la peau et des mains selon les revendications 1 à 11, **caractérisé en ce qu'**il présente comme abrasifs du composant d.) des agents de frottement en matière synthétique à base de polyéthylène ou de polyuréthane, ou des agents de frottement à base de farines de noyaux, d'enveloppes et/ou de coquilles naturelles choisies parmi la farine de coquilles de noix, de coquilles d'amandes, de coquilles de noisettes, de noyaux d'olives, de noyaux d'abricots, de noyaux de cerises et de maïs ou des mélanges de ces farines de coquilles et de noyaux.

13. Agents de nettoyage de la peau et des mains selon les revendications 1 à 12, **caractérisés en ce que** les abrasifs du composant d.) peuvent être obtenus sous forme de produits de polymérisation gonflables à l'eau par polymérisation des composants

    aa.) 55 à 99,95 % en poids de monomères portant des groupes carboxyle monoéthyléniquement insaturés,
    bb.) 0,05 à 5,0 % en poids d'au moins un agent de réticulation,
    cc.) 0 à 40 % en poids d'autres monomères copolymérisables avec a.),
    dd.) 0 à 30 % en poids d'une base de greffage soluble dans l'eau,

et les constituants aa.) à dd.) se complètent à 100 % en poids, où les produits de polymérisation obtenus peuvent éventuellement être post-réticulés au moins une fois.

**14.** Agents de nettoyage de la peau et des mains selon les revendications 1 à 13, **caractérisés en ce qu'**ils sont anhydres.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4335933 A1
- EP 1504081 B1
- WO 0185103 A
- EP 0635260 A
- EP 0141410 A
- US 5824327 A
- US 5444096 A
- DE 3736970
- DE 102007022693

**In der Beschreibung aufgeführte Nicht-Patentliteratur**

- **H. TRONNIER ; J. KRESKEN ; K. JABLONSKI ; B. KOMP.** Haut und Beruf. Grosse Verlag, 1989, 75-108